(19) Europäisches Patentamt
European Patent Office
Office européen des brevets

(11) Veröffentlichungsnummer: **0 161 548**
**B1**

(12) # EUROPÄISCHE PATENTSCHRIFT

(45) Veröffentlichungstag der Patentschrift: **13.06.90**

(51) Int. Cl.$^5$: **C 07 D 475/14** // C07C245/06

(21) Anmeldenummer: **85104916.3**

(22) Anmeldetag: **23.04.85**

(54) **Verbessertes Verfahren zur Herstellung von Riboflavin und neue Zwischenprodukte für dieses Verfahren.**

(30) Priorität: **15.05.84 DE 3417944**

(43) Veröffentlichungstag der Anmeldung:
**21.11.85 Patentblatt 85/47**

(45) Bekanntmachung des Hinweises auf die Patenterteilung:
**13.06.90 Patentblatt 90/24**

(84) Benannte Vertragsstaaten:
**CH DE FR GB LI NL**

(56) Entgegenhaltungen:
**US-A-2 350 376**
**JOURNAL OF THE AMERICAN CHEMICAL SOCIETY, Band 69, Nr. 6, 21. Juni 1947, Seiten 1487-1492, University of Texas, Austin, US; M. TISHLER et al.: "The reaction between o-aminoazo compounds and barbituric acid. A new synthesis of riboflavin"**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Ernst, Hansgeorg, Dr.**
**Bruesseler Ring 38**
**D-6700 Ludwigshafen (DE)**
Erfinder: **Eckhardt, Heinz, Dr.**
**Ruedigerstrasse 7**
**D-6700 Ludwigshafen (DE)**
Erfinder: **Paust, Joachim, Dr.**
**Ringstrasse 3**
**D-6708 Neuhofen (DE)**

**EP 0 161 548 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein verbessertes Verfahren zur Herstellung von Riboflavin (I; Vitamin $B_2$) durch Kondensation eines 4,5-Dimethyl-N-(D)-ribityl-2-phenylazo-anilinderivates (II) mit Barbitursäure (III) in Gegenwart eines sauren Kondensationsmittels in einem organischen Lösungsmittel. Ferner betrifft die Erfindung 4,5-Dimethyl-N-(D)-ribityl-2-(o-alkoxy-phenylazo)-aniline der Formel IIa

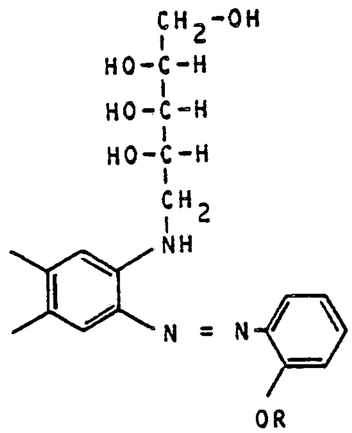

(IIa)

in der R für Alkyl mit 1 bis 4 C-Atomen, insbesondere für Methyl steht.

Die Verbindungen der Formel IIa ermöglichen überaschenderweise eine vorteilhaftere Durchführung des oben beschreibenen Verfahrens.

Dieser letzte Schritt der Ribloflavinsynthese ist — sieht man von der erfindungsgemäßen Verbesserung ab — aus mehreren Veröffentlichungen bekannt. Erstmals beschrieben wurde die Umsetzung von II mit III zu I in J. Am. Chem. Soc, *69* (1947), S. 1487 f. Als Reaktionsmedium wurde hierin insbesondere ein Gemisch aus Eisessig und Dioxan genannt. Da bei diesem Verfahren das relativ teure Lösungsmittel Dioxan in großen Mengen verwendet werden muß und zudem gegen Dioxan Bedenken wegen gesundheitsgefährdender Wirkung bestehen, ist dieses Verfahren für eine Riboflavinherstellung in technischem Maßstab nicht geeignet.

Eine ausführliche Untersuchung über die Eignung verschiedener organischer Säuren als Katalysatoren für die Umsetzung von II mit III wurde von Berezowski et al im J. Gen. Chem. USSR *31* (1961), Seiten 3444 f beschrieben. Als Lösungsmittel wurde siedendes Butanol verwendet. Die hierbei erzielten Ausbeuten von maximal ca. 70% befriedigen bei einem Verfahren im technischen Maßstab nicht, zumal wenn es sich um den letzten Reaktionsschritt eines vielstufigen Verfahrens handelt, und zumal diese Ausbeuten auch nur bei Verwendung von größeren III-Überschüssen erzielt wurden.

Gemäß der CS—A—127 303 wird II mit III in Gegenwart von Eisessig in Butanol oder Dioxan umgesetzt. Die erzielten Ausbeuten betragen maximal 78%.

Aus der JA—A—7737/1963 ist die Umsetzung von II mit III in einem Gemisch aus Alkanolen mit Kp = 80 bis 120°C und Eisessig an $Al_2O_3$ oder gereinigter Diatomeenerde bekannt. Die Ausbeuten betragen 67—69% der Theorie.

Aus der JA—A—10151/1968 ist weiterhin ein Verfahren zur Kondensation von II mit III in einem Gemisch aus einem Lösungsmittel wie Essigsäureethylester und Nitrobenzol oder einem nitrierten Alkan bekannt. Die Ausbeuten betragen 82—84%. Nachteilig an diesem Verfahren sind einerseits zu niedrige Raum-Zeit-Ausbeuten sowie die Notwendigkeit der Mitverwendung von Nitrobenzol u.ä., welches einen sehr hohen Dampfdruck aufweist und äußerst giftig ist.

Aus der CS—A—195 229 ist ein Verfahren zur Herstellung von I aus II und III in einem Gemisch aus Xylol, einem $C_3$—$C_5$-Alkanol, Eisessig und Essigsäureestern bekannt. Die Ausbeuten betragen 86—87,4% an einem relativ reinen I. Nachteilig an diesem Verfahren sind die aufwendige Aufarbeitung des eingesetzten Lösungsmittelgemisches, schlechte Raum-Zeit-Ausbeuten und Schwierigkeiten beim Abfiltrieren des Produktes, da I aus diesem Gemisch in relativ kleinen schwer filtrierbaren Kristallen ausfällt.

Weiterhin ist in der JA—A—3575/1957 ein Verfahren zur Herstellung von I durch Umsetzen von II mit III in einem Gemisch aus einem organischen Lösungsmittel und Eisessig an einem sauren Salz wie $SnCl_2$, $ZnCl_2$, $FeCl_2$, $AlCl_3$ oder $BiCl_3$ als Katalysator bekannt. Man erhält I-Ausbeuten über 80% der Theorie. Nachteilig an diesem Verfahren ist, daß die erwähnten guten Ausbeuten nur erzielt werden, wenn man mit einem Barbitursäureüberschuß von 75 bis 110% arbeitet, daß das erhaltene Riboflavin in jedem Fall einer mit Ausbeuteverlusten verbundenen Nachreinigung unterzogen werden muß und daß die Regeneration der Lösungsmittel und der Metallsalze recht zeit- und kostenaufwendig ist.

Es war daher die Aufgabe der Erfindung, für den oben beschriebenen letzten Schritt der Riboflavinsynthese die Nachteile des Standes der Technik zu überwinden, d.h. eine Möglichkeit zu finden, diesen Schritt auf einfache Weise, in kürzerer Zeit und mit besseren Ausbeuten durchzuführen und dennoch möglichst reines Riboflavin zu erzielen.

Als Azokomponente wird bei der Kondensation zum Riboflavin üblicherweise eine solche Verbindung der Formel II verwendet, in der der Phenylring der Phenylazogruppe nicht substituiert ist.

In dem Verfahren gemäß US—2 350 376 dagegen wurde ein am Phenylring der Phenylazogruppe substituiertes II, nämlich 4,5-Dimethyl-N-(D)-ribityl-2-(p-nitro-phenylazo)-anilin mit Barbitursäure kondensiert.

In J. Am. Chem. Soc. *69* (1947), Seite 1487f wurde die Abhängigkeit der Riboflavinausbeute von den Substituenten $R^1$ und $R^2$ in der Azoverbindung der allgemeinen Formel IV untersucht.

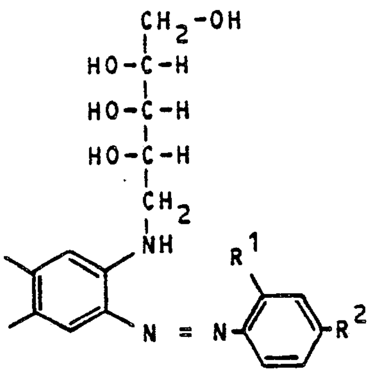

| IV | $R^1$ | $R^2$ |
|---|---|---|
| *a* | H | H |
| *b* | $NO_2$ | H |
| *c* | H | $NO_2$ |
| *d* | Cl | H |
| *e* | H | Cl |
| *f* | H | $OCH_3$ |
| *g* | H | COOH |
| *h* | H | $CH_3$ |
| *i* | $CH_3$ | H |

(IV)

Es zeigte sich, daß die besten Riboflavinausbeuten mit *IVa* ($R^1$=$R^2$=H) erzeilt wurden. Geringfügig schlechtere Ausbeuten erhielt man ausgehend von *IVb* und *IVc.* Deutlich schlechtere Ausbeuten wurden auch mit *IVd, e* und *f* erzielt. Als deutlicher ausgeprägt erwiesen sich die Unterschiede in der Riboflavinausbeute in Abhängigkeit von $R^1$ und $R^2$ bei Einsatz der entsprechenden Tetraacetylverbindungen *Va-f.*

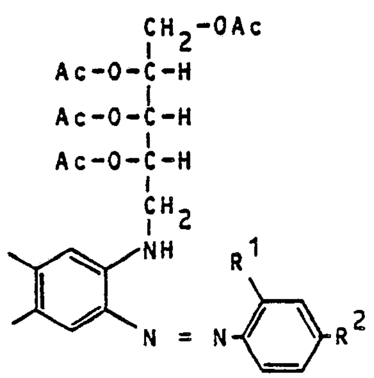

| V | $R^1$ | $R^2$ |
|---|---|---|
| *a* | H | H |
| *b* | $NO_2$ | H |
| *c* | H | $NO_2$ |
| *d* | Cl | H |
| *e* | H | Cl |
| *f* | H | $OCH_3$ |
| *g* | $CH_3$ | $CH_3$ |
| *h* | H | $CH_3$ |

(V)

Hier ist eine klare Abhängigkeit der Ausbeute von der Substitution am Phenylring der Phenylazogruppe zu erkennen, und zwar *Vc* (p-$NO_2$)>*Vb* (o-$NO_2$)>*Va* (unsubstituiert)>*Ve* (p-Cl)>*Vd* (o-Cl)>*Vf* (p-$OCH_3$).

Gemäß Liebigs Ann. Chem. *615* (1958), Seiten 94f wurde die Ausbeute an I durch Einsatz der Azoverbindung *IVg* anstelle von *IVh* um etwa 15% auf etwa 61% gesteigert.

Aus all diesen Befunden geht hervor, daß die Riboflavinausbeute bei Einführung von Substituenten mit −M-Effekt (COOH, $NO_2$) an der Phenylazogruppe steigt, bei Substituenten mit +M-Effekt (Cl, $OCH_3$) dagegen deutlich im Vergleich zum unsubstituierten Aromaten abfällt. Außerdem besteht ein sterischer Effekt: o-Substitutionsprodukte führen (mit Ausnahme von *IVb*) zu erheblich niedrigeren Ausbeuten als die entsprechenden p-Substitutionsprodukte. Deise Befunde konnten in der von uns durchgeführten, als Vergleichsbeispiel 1 zusammengefaßten Versuchsreihe bestätigt werden.

Nach J. Am. Chem. Soc. *69* (loc. cit) liefert das p-$NO_2$-Derivat *Vc* die besten Riboflavinausbeuten. Zur Synthese von *Vc* muß jedoch zunächst die Tetraacetylverbindung *VI* synthetisiert werden und diese anschließend mit p-Nitrophenyldiazoniumsalz gekuppelt werden. Nach Kondensation mit Barbitursäure

müssen dann in einer zusätzlichen Stufe die 4 Acetylgruppen abgespalten werden, bevor man zum Riboflavin gelangt.

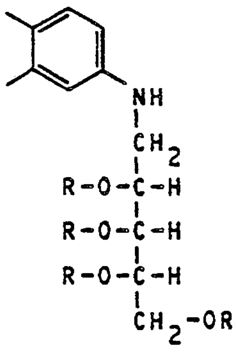

VI: R = Acetyl
VII: R = H

Die direkte Kupplung der nichtacetylierten Verbindung 3,4-Dimethyl-N-ribityl-anilin (VII) mit p-Nitrophenyldiazoniumsalz führte nicht zu einem sauberen Produkt, da der Anteil des für die Herstellung von I unbrauchbaren Isomeren VIII (hier VIIIa) bei Verwendung dieses Diazoniumsalzes besonders hoch war.

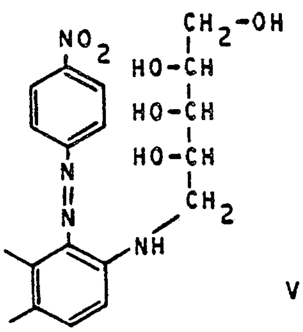

VIIIa

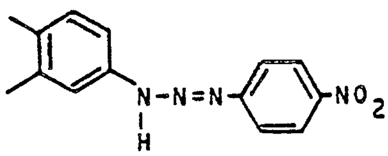

IXa

Außerdem führt die Kupplung von aromatischen Aminen mit elektronenarmen Diazoniumsalzen wie z.B. p-Nitrophenyl-diazoniumsalzen leicht zur Bildung von Triazenen des Typs IXa, die als Verunreinigungen nur schwer abzutrennen sind.

Aus der oben erläuterten Literatur geht also hervor, daß +M-Effekt bedingende Substituenten und o-Substitution am Phenylring der Azokomponente zur Verringerung der Riboflavinausbeute führen.

Es war daher besonders überraschend, daß unter Verwendung der bislang nicht in der Litertur beschreibenen, in o-Position mit einer niederen Alkoxygruppe substituierten Azoverbindung *IIa* besonders gute Ausbeuten erhalten werden (siehe Beispiel 1 und Vergleichsbeispiele 1a bis 1f). Außerdem ist die Bildungsgeschwindigkeit von Riboflavin bei Verwendung von *IIa* deutlich höher als bei den anderen Azoverbindungen (vgl. Beispiel 2 und Vergleichsbeispiele 2a bis 2f).

Ein weiterer Vorteil bei der Verwendung der erfindungsgemäßen 4,5-Dimethyl-N-(D)-ribityl-2-(o-alkoxy-phenylazo)-aniline der Formel IIa ergibt sich aus folgenden Fakten:

Wie oben bereits erwähnt wurde, bildensich bei der Umsetzung von VII mit Phenyldiazoniumsalzen üblicherweise neben den für die I-Herstellung erwünschten, gegebenenfalls substituierten Azoverbindungen der Formel II auch die für die I-Herstellung unbrauchbaren Isomeren VIII,

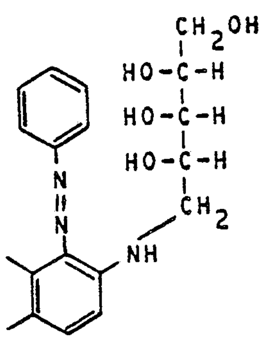

(VIII)

so daß die für die Kondensation mit III eingesetzte Azoverbindung eigentlich ein Gemisch aus gegebenenfalls substituiertem II und VIII ist (vgl. Ullmanns Encyklopädie der technischen Chemie, Band *23*, Verlag Chemie, Weinheim-Bergstraße, 4. Auflage, 1983, Seite 666).

Da das in II enthaltene VIII bei der Kondensation mit Barbitursäure das Isoriboflavin X

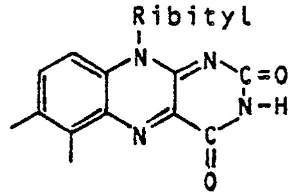

( X)

bildet und dieses einerseits nur schwer abtrennbar ist und andererseits die Wirkung des Riboflavins beeinträchtigt (vgl. Rivlin "Riboflavin", Plenum Press New York, N.Y., 1975, Seiten 318—319), ist est für eine technische Riboflavinsynthese sehr wichtig, den Gehalt an VIII in II so gering wie möglich zu halten.

Überaschenderweise erhält man bei der Umsetzung von VII mit o-Alkoxy-anilindiazoniumsalzen die Azoverbindungen IIa in nahezu isomerenreiner Qualität, die das falsche Isomer nur in Spuren (1 bis 2%) enthalten, wodurch bei der anschließenden Kondensation, die bei dem falschen Isomeren langsamer verläuft, auch praktisch kein Isoriboflavin gebildet wird.

Gegenstand der Erfindung ist daher neben den 4,5-Dimethyl-N-(D)-ribityl-2-(o-alkoxy-phenylazo)-anilinen der Formel IIa ein Verfahren zur Herstellung von Riboflavin der Formel I

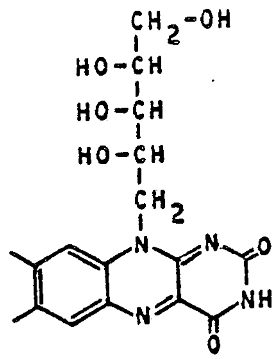

(I)

durch Kondensation eines 4,5-Dimethyl-N-(D)-ribityl-2-phenylazo-anilins der Formel II

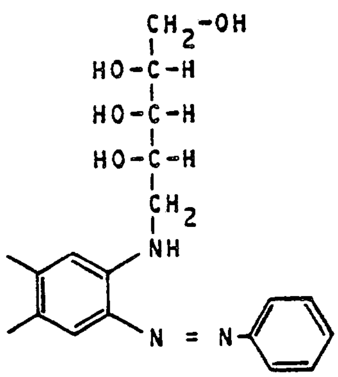

(II)

in dem der Phenylring der Phenylazogruppe in ortho- oder para-Stellung substituiert sein kann, mit Barbitursäure der Formel III

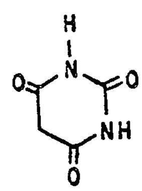

(III)

in Gegenwart eines sauren Kondensationsmittels in einem organischen Lösungsmittels, das dadurch gekennzeichnet ist, daß man 4,5-Dimethyl-N-(D)-ribityl-2-(o-alkyl-phenylazo)-anilin der Formel IIa

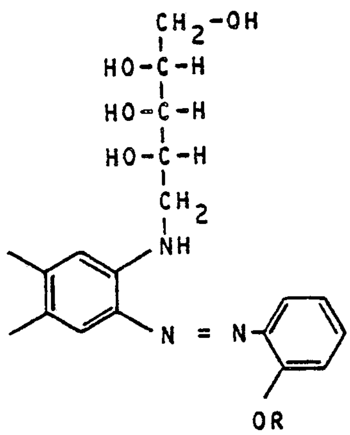

(IIa)

in der R für Alkyl mit 1 bis 4 C-Atomen, insbesondere für Methyl steht, mit Barbitursäure umsetzt.

Die als Ausgansverbindungen für das erfindungsgemäße Verfahren verwendeten neuen 4,5-Dimethyl-N-(D)-ribityl-2-(o-alkoxy-phenylazo)-aniline der Formel IIa erhält man in an sich bekannter Weise durch Umsetzen einer aus o-Alkoxy-anilin mit Natriumnitrit in HCl- oder schwefel-saurer Lösung erhaltenen Lösung des entsprechenden o-Alkoxy-phenyldiazoniumsalzes mit 3,4-Dimethyl-N-ribityl-anilin (vgl. Beispiel 4).

Zweckmäßigerweise nimmt man die erfindungsgemäße Umsetzung von Verbindungen der formel IIa mit III — wie auch bisher schon üblich — in Gegenwart eines inerten Verdünnungs- oder Lösungsmittels vor. Als Lösungsmittel eignen sich vorzugsweise solche, in denen auch das bei der Kondensation entstehende Wasser löslich ist oder zumindest teilweise löslich ist, also Dioxan, Tetrahydrofuran, Dimethylformamid und vor allem die relativ billigen niederen Alkohole mit einem Siedepunkt von 80 bis 150°C, wie Propanol, Isopropanol, n-Butanol, Isobutanol und n-Pentanol. Nach neuesten, nicht vorveröffentlichten Ergebnissen eignen sich besonders auch Acetate mehrwertiger aliphatischer Alkohole, wie Ethylenglykoldiacetat, Butan-1,4-diol-diacetat oder Glycerintriacetat sowie Alkoxyalkanolacetate, wie 2-Ethoxy-ethylacetat, und sogar Alkoxyalkanole, wie 1-Methoxy-2-propanol. Mit besonderem Vorteil verwendet man Ethylenglykoldiacetat oder 1-Methoxy-2-propanol bzw. ein Gemisch aus diesen beiden Lösungsmitteln. Die Menge des Lösungsmittels beträgt im allgemeinen etwa 2 bis 12 Liter pro Kilo II.

Als saure Kondensationsmittel kommen im Prinzip alle bisher in der Literatur für diese Umsetzung beschriebenen Säuren, vorzugsweise schwache organische Säuren wie Eisessig, Propionsäure, Phenylessigsäure, Benzoesäure, Trimethylessigsäure (Pivalinsäure), 2,2-Dimethylbutansäure, 2,2-Dimethylpentansäure und 1-Methyl-cyclopentancarbonsäure in Betracht.

Technisch von besonderer Bedeutung sind, nach neuesten, bisher nicht vorveröffentlichten Erkenntnissen, der besonders billige Eissessig und im Handel erhältliche Gemische synthetischer Säuren, die im wesentlichen gesättigte tertiäre Carbonsäuren enthalten, wie vor allem die sogenannte Versatic®-10-Säure, eine synthetische $C_{10}$-Carbonsäure der Shell Chemie sowie ähnliche Produkte der Firma Esso, die unter dem Namen "Neosäuren" im Handel sind. Genannt seien hiervon beispielsweise die "Neopentansäure", die als Hauptbestandteil Trimethylessigsäure enthält, und die "Neodecansäure", die in ihrer Zusammensetzung der Versatic-10-Säure ähnlich sein dürfte.

Die Menge der Carbonsäure beträgt vorzugsweise etwa 0,5 bis 6 Mol pro Mol IIa, was beispielsweise für Eisessig etwa 0,08 bis 1 kg pro kg IIa und für Versatic-10-Säure 0,2 kg bis 2,7 kg pro kg IIa entspricht.

Es ist ein besonderer Vorteil des erfindungsgemäßen Verfahrens, daß man auch bei Einsatz äquimolarer Mengen von IIa und III deutlich höhere Ausbeuten als bisher erzielt. Diese Ausbeuten betragen etwa 85%, lassen sich aber noch auf weit über 90% erhöhen, wenn man III in einem bis zu 0,3-molaren Überschuß verwendet. Höhere Überschüsse stören nicht, erbringen jedoch keine merklichen Ausbeutesteigerungen mehr.

Die Reaktionstemperaturen liegen bei 80—120°C. Vorzugsweise arbeitet man bei Temperaturen von über 100°C, also bei etwa 100 bis 115°C sowie in einem entsprechend hochsiedenden Lösungsmittel.

Die Aufarbeitung des Reaktionsgemisches kann nach den üblichen Methoden erfolgen, etwa indem man es abkühlen läßt und das auskristallisierte Riboflavin abfiltriert.

Die erfindungsgemaßen 4,5-Dimethyl-N-(D)-ribityl-2-(o-alkoxy-phenylazo)-aniline IIa bilden bei der Kondensation mit Barbitursäure sehr reines Riboflavin in kürzeren Reaktionszeiten und besseren Ausbeuten als unsubstituierte oder anders substituierte Azoverbindungen der allgemeinen Formel IV.

Beispiel 1 und Vergleichsbeispiele 1a bis 1f

Jeweils 25 mmol der aus der folgenden Tabelle I ersichtlichen Azoverbindungen der allgemeinen Formel IV

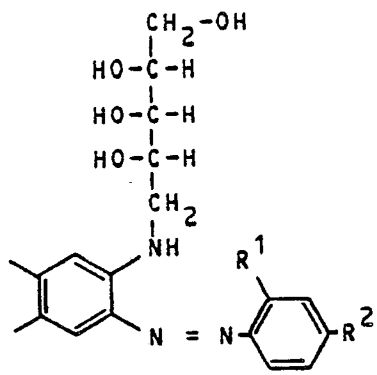

(eingesetzt wurden Isomerengemische mit einem Gehalt an dem Isomeren IV zwischen 90 und 97%; alle Mengenangaben sind bezogen auf die eingesetzte Menge an dem Isomeren IV) wurden mit 3,20 g (25 mmol) Barbitursäure in einem Gemisch aus 100 ml Dioxan und 15 ml Pivalinsäure 16 Stunden (h) unter Rückfluß zum Sieden erhitzt. Anschließend ließ man das Reaktionsgemisch auf Raumtemperatur (RT) abkühlen, saugte die ausgeschiedenen Riboflavinkristalle ab, wusch 2mal mit je 50 ml Metanol (RT), 2mal mit je 80 ml Wasser (80°C) und nochmals 2mal mit je 50 ml Methanol (RT) und trocknete schießlich über Nacht unter vermindertem Druck bei 70°C. Die Ergebnisse sind in der folgenden Tabelle dargestellt. Die Reinheit des Riboflavins wurde durch UV-Messung gemäß Pharmakopoe Europa bestimmt.

Tabelle 1

| Beispiel | Ausgansverb. IV $R^1$ | $R^2$ | Ausbeute [g] | Ausbeute [% d.Th.] | Reinheit [%] |
|---|---|---|---|---|---|
| 1 | $-O-CH_3$ | H | 7,87 | 83,7 | 96,1 |
| Vergleichsbeispiele | | | | | |
| 1a | H | H | 6,56 | 69,8 | 97,7 |
| 1b | H | $-CH_3$ | 6,44 | 68,5 | 97,2 |
| 1c | $-CH_3$ | H | 5,20 | 55,3 | 86,2 |
| 1d | H | -Cl | 5,19 | 55,2 | 92,3 |
| 1e | Cl | H | 4,63 | 49,3 | 90,7 |
| 1f | H | $-O-CH_3$ | 3,72 | 39,6 | 88,0 |

Bemerkt sei noch, daß unter den Bedingungen von Beispiel 1 die Ausbeute an Riboflavin bereits nach 4 Stunden 70% beträgt und die Umsetzung nach 10 Stunden praktisch abgeschlossen war, während bei den Vergleichsversuchen 1a bis 1f die angegebenen Ausbeuten erst nach 16 h erreicht waren.

Beispiel 2 und Vergleichsbeispiele 2a bis 2f

Jeweils 25 mmol der aus der folgenden Tabelle 2 ersichtlichen Azoverbindung der Formel IV wurden mit 3,20 g (25 mmol) Barbitursäure in einem Gemisch aus 100 ml Dioxan und 15 ml Pivalinsäure unter Rückfluß zum Sieden erhitzt. Jeweils nach 2, 4, 7, 10 und 16 Stunden wurden die Reaktionsansätze auf RT abgekühlt, analog Beispiel 1 aufgearbeitet und aus den erhaltenen Riboflavinmengen die in Tabelle 2 angegebenen Ausbeuten errechnet. Die Reinheiten der erhaltenen Ribiflavinproben entsprach etwa den in Beispiel 1 bzw. Vergleichsbeispielen 1a bis 1f angegebenen Reinheiten.

Tabelle 2

| Beispiel | Ausgansverb. IV | | Ausbeute als f (h) nach [%] | | | | |
|---|---|---|---|---|---|---|---|
| | $R^1$ | $R^2$ | 2 h | 4 h | 7 h | 10 h | 16 h |
| 2 | $-O-CH_3$ | H | 58,5 | 70,5 | 78 | 80,5 | 82 |
| Vgl.bsp. | | | | | | | |
| 2a | H | H | 29,5 | 43 | 54,5 | 62 | 70 |
| 2b | H | $-CH_3$ | 27 | 41 | 53 | 61 | 68 |
| 2c | $-CH_3$ | H | 12,5 | 25 | 37 | 47 | 55 |
| 2d | H | Cl | 12,5 | 26 | 38 | 45,5 | 55 |
| 2e | Cl | H | 10 | 23 | 32,5 | 41 | 49 |
| 2f | H | $-O-CH_3$ | 0,5 | 2 | 5 | 20 | 40 |

Beispiel 3

10,0 g (97% ige Reinheit, entsprechend 0,0249 mol) 4,5-Dimethyl-N-(D)-ribityl-2-(o-methoxy-phenylazo)-anilin wurden mit 3,84 g (0,03 mol) Barbitursäure in einem Gemisch aus 40 ml Isobutanol, 7 ml Isobutylacetat und 4,7 ml Eisessig 10 h unter Rückfluß zum Sieden erhitzt. Anschließend ließ man das Reaktionsgemisch auf RT abkühlen, saugte die gebildeten Kristalle ab, wusch sie mit kaltem Methanol und 80°C warmem Wasser und trocknete sie bei 70°C unter vermindertem Druck. Die Ausbeute betrug 8,2 g; entsprechend 87,6% d.Th. Die Reinheit betrug 94% d.Th. (Pharm. Europ.).

Vergleichsbeispiel 3

20,0 g (87% ige Reinheit; entsprechend 0,0485 mol) 4,5-Dimethyl-N-(D)-ribityl-2-phenylazo-anilin wurden mit 7,26 g (0,0567 mol) Barbitursäure in einem Gemisch aus 80 ml Isobutanol, 14 ml Isobutylacetat und 10 ml Eisessig 10 h unter Rückfluß zum Sieden erhitzt. Nach Aufarbeitung entsprechend Beispiel 3 erhielt man 15,4 g Ribiflavin entsprechend 84,5% d.Th. mit einer Reinheit von 93,8% (Pharm. Europ.).

Beispiel 4

a) Herstellung von 4,5-Dimethyl-N-(D)-ribityl-2-(o-methoxyphenylazo]-anilin

58,6 g (0,476 mol) o-Methoxy-anilin wurden in 140 ml Wasser gelöst, die Lösung mit 118 ml konz. Salzsäure versetzt, auf 0°C abgekühlt und bei 0 bis 5°C tropfenweise mit einer Lösung von 33 g Natriumnitrit in 100 ml Wasser versetzt.

100 g (0,39 mmol) 3,4-Dimethyl-N-(D)-ribityl-anilin wurden in 400 ml 1 n Salzsäure gelöst, die Lösung mittels 30% iger wäßriger Natriumformiatlösung auf einen pH-Wert von 3 eingestellt und anschließend innerhalb 1 h in die hergestellte Lösung des Diazoniumsalzes eingetropft. Das Gemisch wurde 3 h bei RT und 30 min bei 5°C nachgerührt, die gebildeten Kristalle abgesaugt und der Filterkuchen 4mal mit je 500 ml Wasser (80°C) gewaschen. Eine Probe des wasserfeuchten Filterkuchens wurde getrocknet: das NMR-Spektrum zeigte ein Verhältnis der isomeren Azoverbindungen von 98:2. Die Hauptmenge wurde in 350 ml Ethanol gelöst, die Lösung in der Hitze mit 400 ml Wasser versetzt und über Nacht stehen gelassen. Die gebildeten Kristalle wurden abgesaugt, mit 250 ml eines Gemisches aus Ethanol und Wasser (1:1) gewaschen und bei +70°C unter vermindertem Druck getrocknet. Die Auswaage betrug 109,5 g, entsprechend 72,2% d.Th. Fp: 142 bis 144°C. Im NMR-Spektrum ist nur 1 Isomeres zu erkennen.

b) Herstellung von Riboflavin

10,8 g des gemäß a) erhaltenen 4,5-Dimethyl-N-(D)-ribityl-2-(o-methoxy-phenylazo)-anilins (97% ig; entsprechend 0,0269 mol) und 4,4 g Barbitursäure wurden in einem Gemisch aus 35 ml Dioxan und 25 ml Versatic®-10-Säure 9 h unter Rühren und Rückfluß zum Sieden erhitzt. Das Reaktionsgemisch wurde auf RT abgekühlt, die gebildeten Kristalle abgesaugt, 2mal mit je 25 ml Methanol, 3mal mit je 60 ml Wasser (80°C) und nochmals 2mal mit je 25 ml Methanol gewaschen und über Nacht bei +70°C unter vermindertem Druck getrocknet. Die Auswaage betrug 9,46 g Ribiflavin entsprechend 93,5% d.Th. Reinheit: 94,7% (Pharm. Europ.).

Beispiel 5

10,8 g 4,5-Dimethyl-N-(D)-ribityl-2-(o-methoxy-phenylazo)-anilin (97% ig) entsprechend 0,0269 mol) und 4,4 g Barbitursäure wurden in einem Gemisch aus 40 ml 1-Methoxy-propanol-(2) und 25 ml Versatic®-10-Säure 9 h bei 120°C gerührt. Nach Aufarbeitung analog Beispiel 5 erhielt man 9,46 g Riboflavin, entsprechend 93,5% d.Th. Reinheit: 92,2% (Pharm. Europ.).

Beispiel 6

10,8 g 4,5-Dimethyl-N-(D)-ribityl-2-(o-methoxy-phenylazo)-anilin (97% ig; entsprechend 0,0269 mol) und 4,4 g Barbitursäure wurden in einem Gemisch aus 50 ml Isobutanol und 25 ml Versatic®-10-Säure 9 h unter Rühren und Rückfluß zum Sieden erhitzt. Dann ließ man das Reaktionsgemisch auf RT abgekühlen, saugt die gebildeten Kristalle ab, wusch sie 2mal mit je 20 ml Isobutanol, 2mal mit je 25 ml Methanol und 3mal mit je 60 ml Wasser (80°C) und trocknete sie über Nacht bei +70°C unter vermindertem Druck. Auswaage: 9,73 Riboflavin, entsprechend 96,2% d.Th. Reinheit: 94,6% (Pharm. Europ.).

**Patentansprüche**

1. Verbessertes Verfahren zur Herstellung von Riboflavin der Formel I

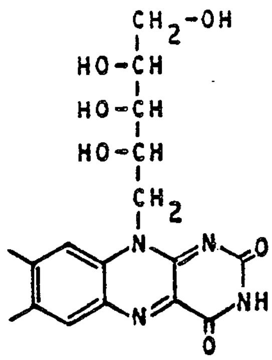

(I)

durch Kondensation eines 4,5-Dimethyl-N-(D)-ribityl-2-phenylazo-anilins der Formel II

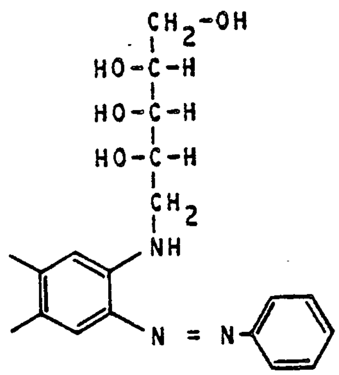

(II)

in dem der Phenylring der Phenylazogruppe in ortho- oder para-Stellung substituiert sein kann, mit Barbitursäure der Formel III

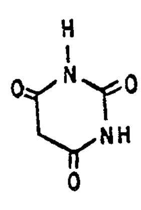

(III)

in Gegenwart einer schwachen organischen Säure in einem organischen Lösungsmittel, dadurch gekennzeichnet, daß man 4,5-Dimethyl-N-(D)-ribityl-2-(o-alkoxy-phenylazo)-anilin der Formel IIa

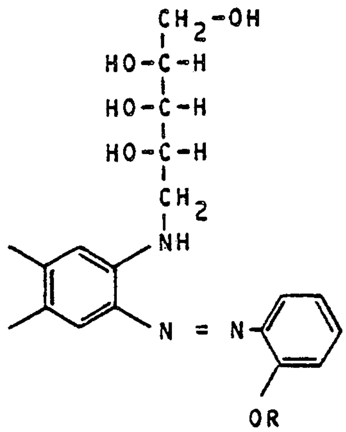

(IIa)

in der R für Alkyl mit 1 bis 4 C-Atomen, insbesondere für Methyl steht, mit Barbitursäure umsetzt.

2. 4,5-Dimethyl-N-(D)-ribityl-2-(o-alkoxy-phenylazo)-aniline der Formel IIa

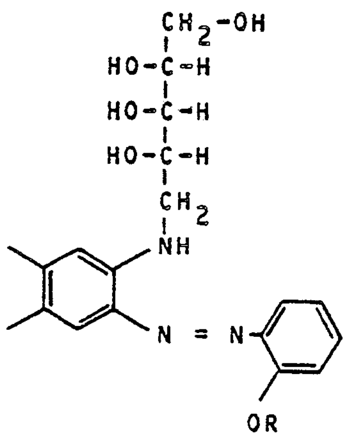

(IIa)

in der R für Alkyl mit 1 bis 4 C-Atomen steht.

3. 4,5-Dimethyl-N-(D)-ribityl-2-(o-methoxy-phenylazo)-anilin.

## Revendications

1. Procédé amélioré pour la préparation de riboflavine de formule I

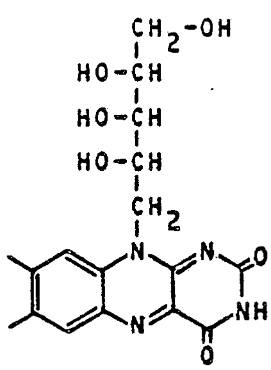

(I)

par condensation d'une 4,5-diméthyl-N-(d)-ribityl-2-phénylazo-aniline de formule II

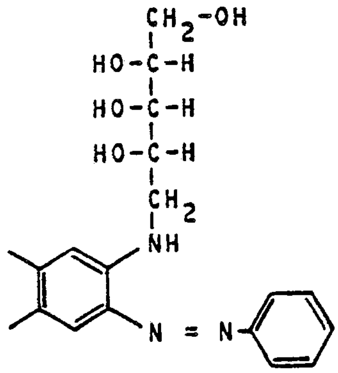

(II)

dans laquelle le noyau phényle du groupe phénylazo peut être substitué en position ortho ou para, avec de l'acide barbiturique de formule III

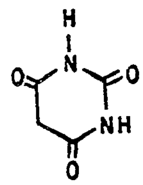

(III)

en présence d'un acide organique faible, dans un solvent organique, ce procédé étant caractérisé par le fait que l'on fait réagir avec l'acide barbiturique de la 4,5-diméthyl-N-(d)-ribityl-2-(o-alcoxy-phénylazo)-aniline de formule IIa

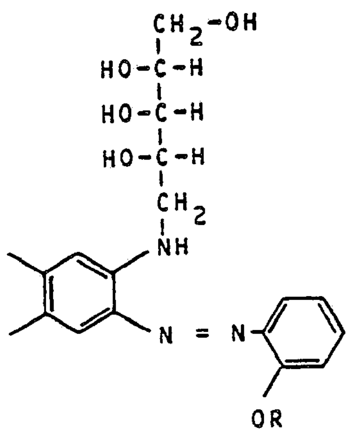

(IIa)

dans laquelle R est mis pour alkyle à 1 à 4 atomes C, en particulier pour méthyle.

2. 4,5-diméthyl-N-(d)-ribityl-2-(o-alcoxy-phénylazo)-anilines de formule IIa

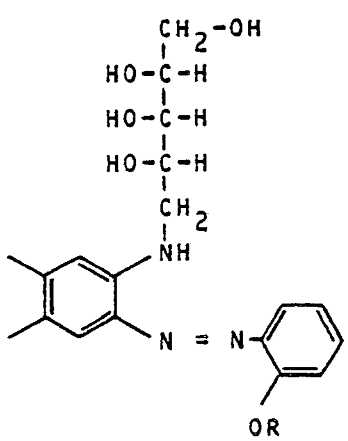

(IIa)

dans laquelle R est mis pour alkyle à 1 à 4 atomes C.

3. 4,5-Dimethyl-N-(d)-ribityl-2-(o-méthoxy-phénylazo)-aniline.

## Claims

1. An improved process for the preparation of riboflavin of the formula I

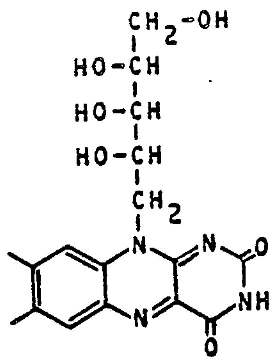

(I)

by condensation of 4,5-dimethyl-N-(D)-ribityl-2-phenylazoaniline of the formula II

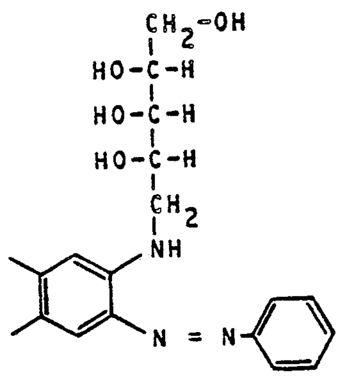

(II)

where the phenyl ring of the phenylazo group can be substituted in the ortho- or para-position, with barbituric acid of the formula III

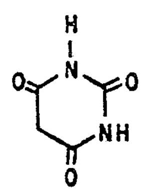

(III)

in the presence of a weak organic acid in an organic solvent, wherein a 4,5-dimethyl-N-(D)-ribityl-2-(o-alkoxyphenylazo)-aniline of the formula IIa

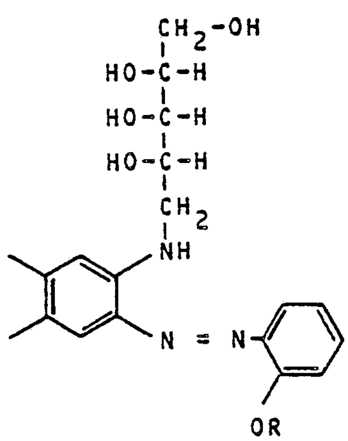

(IIa)

where R is alkyl of 1 to 4 carbon atoms, in particular methyl, is reacted with barbituric acid.

2. A 4,5-dimethyl-N-(d)-ribityl-2-(o-alkoxyphenylazo)-aniline of the formula IIa

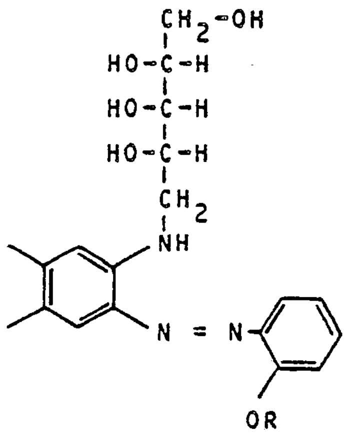

(IIa)

where R is alkyl of 1 to 4 carbon atoms.

3. 4,5-Dimethyl-N-(d)-ribityl-2-(o-methoxyphenylazo)-aniline.